# EUROPEAN PATENT APPLICATION

(11) **EP 4 840 414 A1**
(43) Date of publication of application: **18.11.2026**
(21) Application number: 25176259.7
(22) Date of filing: 14.05.2025
(51) Int. Cl.: A61B 18/18

(54) **LIGHT-BASED SKIN TREATMENT APPARATUS, AND COMPUTER PROGRAM PRODUCT**

(71) Applicant: Koninklijke Philips N.V., 5656 AE Eindhoven (NL)
(72) Inventor: FLINSENBERG, Ingrid Christina Maria, Eindhoven (NL); KINGMA, Harmen Andries, Eindhoven (NL)
(74) Representative: Philips Intellectual Property & Standards

(57) **Abstract**

There is provided a light-based skin treatment device. The light-based skin treatment device comprises a light source, a treatment aperture, and a first temperature sensor. The light source is adapted to generate treatment light. The treatment aperture is arranged to allow passage of the treatment light to irradiate a treatment area of skin with the treatment light. The first temperature sensor is adapted to generate a first temperature signal representative of a first temperature of a first portion of the treatment area. The first temperature sensor is adapted to sense the first temperature of the first portion via the treatment aperture.

## Description

### FIELD OF THE INVENTION

The invention relates to a light-based skin treatment device, such as an intense pulsed light (IPL) device. The invention further relates to a computer program product that makes use of an output signal of the light-based skin treatment device.

### BACKGROUND OF THE INVENTION

Photo-epilation is well known for hair removal and hair growth reduction. Home-use consumer devices are commercially available, such as the Lumea (TM) of Philips (TM). Home-use devices typically use Intense Pulsed Light technology (IPL) from e.g. a Xenon flash lamp at a relatively low fluence (up to 6.5 J/cm²), as compared to professional devices for permanent photo-epilation, that use fluences in excess of 10 J/cm².

IPL technology uses a high-powered, hand-held, flash lamp to deliver an intense, visible, broad-spectrum pulse of light, generally in the visible spectral range of 400 to 1200 nm. Cutoff filters are for example used to selectively filter out shorter wavelengths, especially potentially damaging ultra violet light. The resulting light has a spectral range that targets specific structures and chromophores, in particular the melanin pigment in hair.

The light absorbed by melanin in the hair and hair matrix generates heat that puts the hair follicles in a sleep state. When the treatment is repeated in intervals of 2 to 4 weeks, a long-lasting hair reduction result is obtained.

### SUMMARY OF THE INVENTION

However, many users struggle to move the light-based skin treatment device along the body part to provide the treatment light in a uniform way. There is no visible difference between the skin that was recently treated with the treatment light and untreated skin. As a result, some parts of the skin may be overtreated, whereas other parts of the skin may be undertreated. Overtreating the skin may lead to discomfort or even pain, whereas undertreating the skin may lead to a poor treatment result.

It is an objective of the invention to provide improved treatment with the light-based skin treatment device.

According to a first aspect, there is provided a light-based skin treatment device. The light-based skin treatment device comprises a light source, a treatment aperture, and a first temperature sensor. The light source is adapted to generate treatment light. The treatment aperture is arranged to allow passage of the treatment light to irradiate a treatment area of skin with the treatment light. The first temperature sensor is adapted to generate a first temperature signal representative of a first temperature of a first portion of the treatment area. The first temperature sensor is adapted to sense the first temperature of the first portion via the treatment aperture.

Because the first temperature sensor is adapted to sense the first temperature via the treatment aperture, the first temperature signal represents the temperature of the skin facing the treatment aperture. This way, the first temperature signal provides improved information about whether the light-based skin treatment device at a certain position has irradiated a skin portion facing the treatment aperture with the treatment light or not. Irradiation with the treatment light causes an increase of the temperature of the skin. Also, when moving the light-based skin treatment device after irradiating the treatment area, the first temperature signal provides accurate information when the treatment aperture faces a next, untreated skin portion. If the first temperature signal changes from representing a high temperature to a low temperature, the treatment aperture is moved from a position facing a treated skin portion to a position facing an untreated skin portion. Because the first temperature signal is representative of the temperature of the skin portion in the treatment area, the device is able to detect treated and untreated skin portions more accurately. As a result, overtreatment of the skin is reduced while avoiding large gaps between irradiated portions of the skin. This way, improved treatment with the light-based skin treatment device is obtained.

The light source is adapted to generate treatment light. For example, the light source is adapted to provide light pulses, also referred to as flashes. For example, the light source is adapted to generate light at a high intensity for a short duration, such as for less than 100 ms or less than 50 ms or less than 10 ms or less than 8 ms. The intensity of the light pulse is high enough to perform treatment on the skin. Such treatment is, for example, hair removal, or photo-rejuvenation, or vein treatment, or acne treatment. For example, the light source comprises one or more optical filters. For example, the light source comprises an optical filter to prevent light with a potentially damaging wavelength, such as UV light, from being transmitted to the skin. For example, the light source comprises a Xenon flash lamp, or a laser, or multiple lasers, a LED, or multiple LEDs. For example, the light source is adapted to provide treatment light with wavelengths in the range of 530-1200 nanometers.

The treatment aperture is arranged to allow passage of the treatment light to irradiate a treatment area of skin with the treatment light. For example, the treatment aperture comprises an opening facing the skin when the light-based skin treatment device is operational use. For example, the light source is arranged in a reflector adapted to reflect the treatment light. The treatment aperture is an opening in the reflector. For example, the treatment aperture is a single aperture, or comprises multiple apertures. For example, the treatment aperture has a square shape, or a rectangular shape, or a round shape, or an oval shape. For example, during operational use of the light-based skin treatment device, an edge of the treatment aperture is in contact with the skin.

The treatment area of the skin is a portion of the skin that is irradiated with treatment light via the treatment aperture while the light-based treatment device remains at a certain position relative to the skin. For example, the shape and/or the size of the treatment area is the same as the shape and/or size of the treatment aperture. In another example, there is a difference between the shape and/or the size of the treatment area and the shape and/or size of the treatment aperture. For a user to cover a whole body region, multiple treatment areas are irradiated with treatment light. A body region is, for example, a leg, or an arm, or the bikini line, or the chest, or the back, or the face, or an arm pit.

The first temperature sensor is adapted to generate the first temperature signal representative of the first temperature of a first portion of the treatment area. For example, the first temperature sensor is a non-contact sensor that is adapted to generate the first temperature signal while being at a distance from the first portion. For example, the first temperature signal is an electrical signal or an optical signal. For example, the first temperature sensor is adapted to sense the temperature of a small area, such as a spot. In this example, the first portion of the treatment area is a small area, such as a spot. For example, the first temperature sensor is adapted to sense the temperature of a larger area, such as a surface area covering a quarter or a half or more of the width of the treatment area. In this example, the first portion of the treatment area is the larger area. In this example, the first temperature signal may represent an average temperature or a maximum temperature or a minimum temperature of the larger area. For example, the first temperature signal is representative of the first temperature as a single value. For example, the first temperature signal is representative of the first temperature as a range of values across the first portion.

In an embodiment, the first temperature sensor is arranged outside a path of the treatment light.

According to this embodiment, the first temperature sensor does not block any of the treatment light from reaching the treatment area, because the first temperature sensor is outside the path of the treatment light. As a result, more treatment light is able to reach the skin. In addition, as the first temperature sensor is not exposed to the treatment light, the first temperature sensor is heated less during operational use of the light-based skin treatment device compared to a temperature sensor that is exposed to the treatment light. This improves the accuracy of the first temperature sensor during operational use of the light-based skin treatment device.

In an embodiment, the first temperature sensor is arranged to receive the electromagnetic radiation from the first portion via a radiation path through the treatment aperture.

According to this embodiment, the first temperature sensor is able to generate the first temperature signal based on the received electromagnetic radiation. The electromagnetic radiation propagates along the radiation path from the treatment area via the treatment aperture to the first temperature sensor. This way, there is no need for the first temperature sensor to be in the path of the treatment light, preventing the treatment light being blocked by the first temperature sensor from reaching the treatment area. For example, the first temperature sensor is arranged to receive the electromagnetic radiation from the first portion via the radiation path that is at an obtuse angle with the treatment area. For example, a surface of the first temperature sensor for receiving the electromagnetic radiation is arranged perpendicular to the first radiation path that is at the obtuse angle with the treatment area. This way, the first temperature sensor is able to receive the electromagnetic radiation via the radiation path.

In an embodiment, the light-based skin treatment device comprises a second temperature sensor adapted to generate a second temperature signal representative of a second portion of the treatment area. The second temperature sensor is adapted to sense the second temperature of the second portion via the treatment aperture.

According to this embodiment, the second temperature sensor generates the second temperature signal representative of the second temperature of the second portion of the treatment area. The second portion of the treatment area is different from the first portion of the temperature. When moving the light-based skin treatment device from a position at which the light source irradiated a first treatment area to a next position at which the light source is able to irradiate a next treatment area, the first temperature signal and the second temperature signal are different from each other over time. Because the first temperature sensor and the second temperature sensor sense different portions of the treatment area (i.e., the first portion and the second portion respectively) the first temperature sensor may sense the next, untreated treatment area, before the second temperature sensor senses the next, untreated area, or vice versa. Based on the difference in time, a movement direction, a displacement and/or a speed of the light-based skin treatment device can be determined based on the first temperature signal and the second temperature signal.

In an embodiment, the light-based skin treatment device comprises a processor configured to generate, after irradiation of the treatment area with the treatment light, an output signal representative of a position of the light-based skin treatment device relative to the skin based on the first temperature signal and the second temperature signal.

According to this embodiment, the processor determines a position of the light-based skin treatment device. The position comprises, for example, a displacement and/or a direction of movement and/or a speed. For example, the processor is configured to determine, based on the first temperature signal and the second temperature signal, whether the light-based skin treatment device has displaced sufficiently along the skin to cause the treatment aperture to face a next, substantially untreated, treatment area.

In an embodiment, the light-based skin treatment device comprises a displacement sensor adapted to generate a displacement signal representative of a displacement of the light-based skin treatment device relative to the skin. The processor is configured to determine the output signal based on the displacement signal, the first temperature signal, and the second temperature signal.

According to the embodiment, the processor is configured to determine the position based on the displacement signal, the first temperature signal, and the second temperature signal. By making use of these different signals, the position is determined with improved accuracy. For example, the displacement sensor may not represent the position accurately. This may happen, for example, in case the user moves the light-based skin treatment device in a certain way, such as moving temporarily away from the skin, or in contamination of the skin deteriorate the measurement by the displacement sensor. Such contamination is, for example, an oily skin, or sweaty spots, or skin shine. However, when the first temperature signal and the second temperature signal indicate the light-based skin treatment device is at the edge of the previous treatment area, the processor may calibrate the displacement sensor. The processor may perform this calibration, because an absolute position of the light-based skin treatment device is known, i.e., the edge of the previous treatment area. After the calibration, the displacement sensor represents the position accurately again. For example, the displacement sensor is an optical displacement sensor, such as a mouse sensor, or an optical tracking sensor.

**In** an embodiment, the light-based skin treatment device comprises a movement sensor adapted to generate a movement signal representative of a movement of the light-based skin treatment device. The processor is configured to generate the output signal based on the movement signal, the first temperature signal, and the second temperature signal.

According to this embodiment, the processor is configured to determine the position based on the movement signal, the first temperature signal, and the second temperature signal. By making use of these different signals, the position is determined with improved accuracy. For example, the movement sensor may not represent the position accurately. This may happen, for example, in case the user moves the light-based skin treatment device in a certain way, such as with abrupt movements, or in case the user moves the body region that is being treated. For example, the user moves her leg to a different position. However, when the first temperature signal and the second temperature signal indicate the light-based skin treatment device is at the edge of the previous treatment area, the processor may calibrate the movement sensor. The processor may perform this calibration, because an absolute position of the light-based skin treatment device is known, i.e., the edge of the previous treatment area. After the calibration, the movement sensor represents the position accurately again. For example, the displacement sensor comprises an accelerometer, or a gyroscope, or an inertia measurement unit (IMU).

In an embodiment, the processor is configured to control the light source to generate a flash of treatment light based on the first temperature signal and the second temperature signal.

According to the embodiment, the first temperature signal and the second temperature signal indicate to the processor that the treatment aperture faces a treatment area that was not irradiated with treatment light before, or the light-based skin treatment device has moved sufficiently away from the previous, irradiated, treatment area to allow safe irradiation of the skin with the treatment light. This way, the first temperature sensor and the second temperature sensor provide safety by preventing the same portion of skin to be irradiated multiple times. For example, the user is able to indicate via a user interface, such as a trigger or a button, that the user desires the light-based skin treatment device to irradiate treatment light. Only when the processor determines, based on the first temperature signal and the second temperature signal, that it is safe to generate the treatment light, the processor controls the light source to generate the flash of treatment light.

In an embodiment, the processor is configured to generate an output signal based on the first temperature signal and the second temperature signal. The output signal is representative of the movement of the light-based skin treatment device. The output signal is adapted to cause a display to display a graphical representation of the movement of the light-based skin treatment device.

According to this embodiment, the graphical representation shows to the user information about the movement of the light-based skin treatment device. The information helps the user to move the light-based skin treatment device along the skin. For example, the graphical representation represents the path along which the light-based skin treatment device was moved by the user, or the distance between consecutive treatment areas, or the distance between consecutive flashes of treatment light. For example, based on the graphical representation, the user is guided to move the light-based skin treatment device slower or faster.

In an embodiment, at least one of the first portion and the second portion comprises a center portion of the treatment area.

According to the embodiment, at least one of the first portion and the second portion comprises the center portion of the treatment area. The center portion is a portion of the treatment area that does not have an edge portion of the treatment area. Because the first portion and/or the second portion comprises the center portion, a substantial part of the irradiated treatment area still faces the treatment aperture even when both the first temperature signal and the second temperature signal indicate a low temperature. This arrangement helps when it is desired to have an overlap between adjacent treatment areas. This way, the first temperature signal and the second temperature signal indicate the light-based skin treatment device has moved to the next position at which the next flash of treatment light is to be delivered. For example, the first temperature sensor and the second temperature sensor arranged to have a distance between the first portion and the second portion to be less than 95%, or less than 90% or less than 75%, or 50% of the length of the treatment aperture.

In an embodiment, the first portion comprises a first edge portion of the treatment area. The second portion comprises a second edge portion of the treatment area, opposite to the first edge portion.

According to this embodiment, the first portion and the second portion have opposing edge portions of the treatment area. As a result, when moving the light-based skin treatment device after irradiating the treatment area, and the first temperature signal and the second temperature signal go from a high value to a low value, the treatment aperture faces an untreated skin portion closely adjacent to the irradiated treatment area. This allows the skin to be irradiated with treatment light with no or only a minimum gap between irradiated portions of the skin.

In an embodiment, the light-based skin treatment device comprises a third temperature sensor adapted to generate a third temperature signal representative of a third portion of the treatment area. The third portion of the treatment area comprises a center portion of the treatment area.

According to the embodiment, the third temperature sensor senses the temperature of a center portion. The center portion is between the first edge portion and the second edge portion. When moving the light-based treatment device after irradiating the treatment area, the timing at which the first temperature sensor, the second temperature sensor, and the third temperature sensor sense a change from a high temperature to a low temperature is different. Based on the difference of the three temperature signals, improved information about the movement of the light-based skin treatment device along the skin is provided.

In an embodiment, the first temperature sensor comprises an infrared sensor or a radiance sensor.

Both an infrared sensor and a radiance sensor are able to detect the temperature of a surface while arranged at a distance from that surface. By using an infrared sensor or a radiance sensor as the first temperature sensor, the first temperature sensor is able to sense the temperature of the first portion while allowing treatment light to reach the first portion.

In an embodiment, the light-based skin treatment device is an Intense Pulsed Light device adapted for photo-epilation.

According to this embodiment, the Intense Pulsed Light (IPL) device provides flashes of treatment light to the skin. The flashes cause depilation. For example, the flashes cause hair follicles to stop generating hairs or to generate hairs in a reduced tempo.

In a second aspect of the invention, there is provided a computer program product comprising instructions, which when executed by a processor, cause a method to be carried out. The method comprises receiving the output signal from the light-based skin treatment device. The method comprises causing the graphical representation representative of the movement of the light-based skin treatment to be displayed on the display.

According to the second aspect, the user of the light-based skin treatment device is informed via the graphical representation about the movement of the light-based skin treatment device. As the movement information is based on the first temperature signal and the second temperature signal, the movement information is representative about whether parts of the skin are treated. This guides the user to move the light-based skin treatment along the skin to cover the body region in an improved way with reduced risk of over-treatment.

### BRIEF DESCRIPTION OF THE DRAWINGS

Exemplary embodiments will now be described, by way of example only, with reference to the following figures, in which:
FIG. 1 depicts a light-based skin treatment device according to a first embodiment of the invention;
FIGs. 2A and 2B depict a detail of the first embodiment;
FIG. 3 depicts the temperature as sensed by the first temperature sensor according to the first embodiment;
FIG. 4 depicts the light-based skin treatment device according to a second embodiment;
FIG. 5 depicts the temperatures as sensed by the first and second temperature sensors according to the second embodiment;
FIG. 6 depicts a processor according to a third embodiment;
FIG. 7 depicts a light-based skin treatment device according to a fourth embodiment;
FIG. 8 depicts part of a light-based skin treatment device according to a fifth embodiment;
FIG. 9 depicts part of a light-based skin treatment device according to a sixth embodiment;

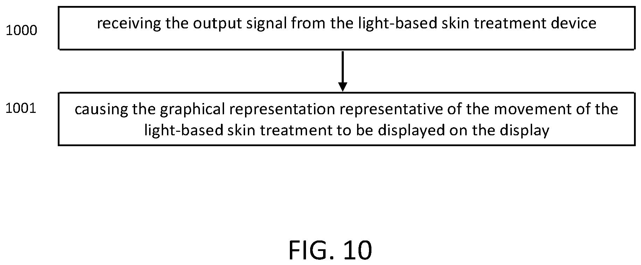

FIG. 10 depicts a method according to a seventh embodiment.

### DETAILED DESCRIPTION OF EMBODIMENTS

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

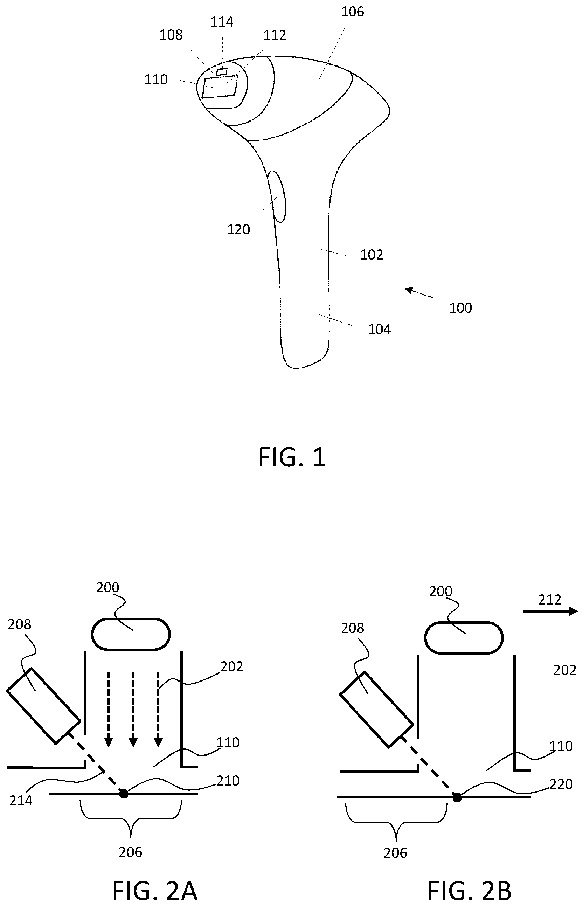

FIG. 1 depicts a light-based skin treatment device 100 according to a first embodiment of the invention. In the first embodiment, the light-based skin treatment device 100 is an Intense Pulsed Light, IPL, device. The IPL device is adapted for photo-epilation. The light-based skin treatment device 100 is adapted for use on the skin of a subject, e.g. a person or an animal. The light-based skin treatment device 100 comprises a housing 102 that has a handle portion 104 and a head portion 106. The handle portion 104 is shaped to enable the user to hold the light-based skin treatment device 100. The head portion 106 has a head end 108 that is to be placed into contact with the subject in order for the light-based skin treatment device 100 to perform a treatment operation on the skin of the subject.

The light-based skin treatment device 100 comprises a light source 200, as shown in FIG. 2, adapted to provide treatment light 202 to the skin. The light source 200 is arranged inside the head portion 106. The light-based skin treatment device 100 performs the treatment operation by providing pulses of the treatment light 202 to the skin via a treatment aperture 110. The treatment aperture 110 is arranged in or on the housing 102 so that the treatment aperture 110 can be placed adjacent to or on (i.e. in contact with) the skin of the subject. Optionally, an IPL window 112 is arranged in the treatment aperture 110. The intensity of the light pulses from the light source 200 are high enough to perform the treatment operation on the skin or body part adjacent to the treatment aperture 110.

The light-based skin treatment device 100 comprises a user control 120 adapted to be operated by the user to activate the light-based skin treatment device 100. When the user activates the light-based skin treatment device 100 via the user control 120, the light-based skin treatment device 100 is able to perform the treatment operation. The user control 120 may be in the form of a switch, a button, a touch pad, etc.

Optionally, the light-based skin treatment device 100 has a sensor window 114. The sensor window 114 is transparent to allow an optical sensor to sense the skin through the sensor window 114. For example, a tone sensor is arranged behind the sensor window 114. The skin tone sensor is adapted to sense a skin tone of the skin facing the sensor window 114. The skin tone sensor generates skin tone data. The light-based skin treatment device 100 is adapted to adjust the skin treatment based on the skin tone data.

FIGs. 2A and 2B depict a detail of the first embodiment. The light-based skin treatment device 100 comprises the light source 200 and the treatment aperture 110. The light source 200 is adapted to generate the treatment light 202. The treatment aperture 110 is arranged to allow passage of the treatment light 202 to irradiate a treatment area 206 of skin with the treatment light 202. The light-based skin treatment device 100 comprises a first temperature sensor 208. The first temperature sensor 208 is adapted to generate a first temperature signal representative of a first temperature of a first portion 210 of the treatment area 206. The first temperature sensor 208 is adapted to sense the first temperature of the first portion 210 via the treatment aperture 110.

The first portion 210 is represented as a point. In this embodiment, the first temperature sensor 208 is adapted to sense the temperature of only a small part of the treatment area 206, so the first portion 210 is small compared to the rest of the treatment area 206. In another embodiment, the first temperature sensor 208 is adapted to sense the temperature of a substantial portion of the treatment area 206, so the first portion 210 is a substantial part of the treatment area 206, such as 10% or 25% or 50% or more than 50% of the treatment area 206.

When the user operates the light-based skin treatment device 100, the light source 200 generates the treatment light 202. The treatment light 202 irradiates the treatment area 206 via the treatment aperture 110. As a result of the irradiation with the treatment light 202, the temperature of the treatment area 206 increases. As the first portion 210 is part of the treatment area 206, the first temperature sensor 208 senses the increase in temperature and generates the first temperature signal accordingly.

After the user operated the light-based skin treatment device 100 to irradiate the treatment area 206, the user moves the light-based skin treatment device 100 in the direction of arrow 212 to a next portion of the skin that is to be treated. While the light-based skin treatment device 100 is moving, the first temperature sensor 208 senses the temperature of a portion 220 of the skin outside the treatment area 206 that was irradiated with the treatment light 202. The portion 220 has a lower temperature than the temperature of the first portion 210 in the irradiated treatment area 206, because the portion 220 has not yet been irradiated with the treatment light 202.

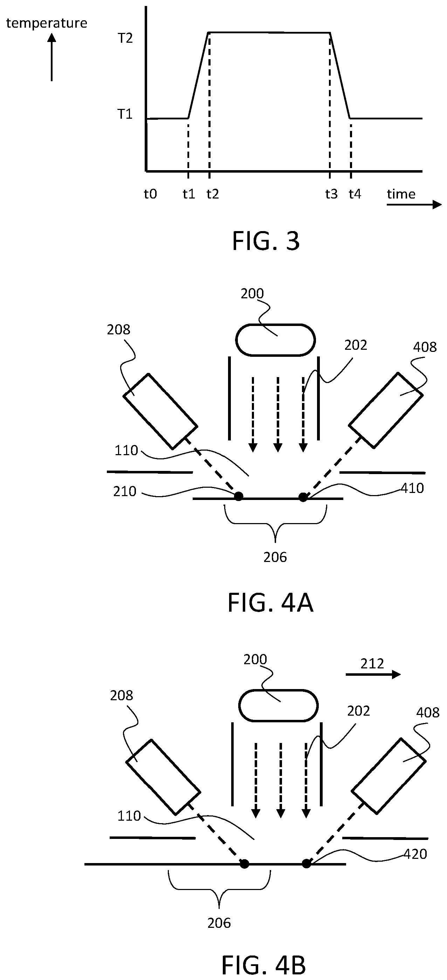

FIG. 3 depicts the temperature as sensed by the first temperature sensor 208 according to the first embodiment. At the beginning, at time t0, the light-based skin treatment device 100 is held by the user at the treatment area 206, and the first temperature sensor 208 senses the temperature of the first portion 210. At t0, the treatment area 206 is not yet irradiated with treatment light 202, so the first temperature signal is representative of a low temperature T1. Then, around t1, the light source 200 starts to generate the treatment light 202, and the treatment area 206 is irradiated with the treatment light 202. As a result, the temperature as sensed by the first temperature sensor 208 increases from the low temperature T1 at t1 to the higher temperature T2 at t2. After the treatment area 206 is irradiated with the treatment light 202, the light-based skin treatment device 100 is moved in the direction of arrow 212. As a result, the first temperature sensor 208 senses the high temperature T2 till time t3. At time t3, first temperature sensor 208 starts to sense the skin outside the treatment area 206 that was irradiated with the treatment light 202. As a result, from time t3, the temperature as sensed by the first temperature sensor 208 starts to decline till it reaches the low temperature T1 at time t4. From time t4, the first temperature sensor 208 senses the temperature of skin that is not irradiated by the treatment light 202, until the light source 200 generates the next irradiation with treatment light 202.

Even though FIG.3 shows a constant temperature T1 from times t0-t1 and t4 onwards, and a constant temperature T2 between times t2-t3, there may be variation of the temperature around temperature T1 and T2 respectively. Temperature T1 may be in the range of 25-33 degrees Celsius, which is a typical skin temperature range. Temperature T2 is, for example, at least 1, or at least 1.5 or at least 2 degrees Celsius higher than temperature T1.

As shown in FIG 2A, the first temperature sensor 208 is arranged outside a path of the treatment light 202. The first temperature sensor 208 is arranged to receive the electromagnetic radiation from the first portion 210 via a radiation path 214 through the treatment aperture 110. The radiation path 214 is indicated with the dashed line.

For example, the first temperature sensor 208 comprises an infrared sensor or a radiance sensor. For example, the first temperature sensor 208 comprises an infrared camera or a microbolometer. For example, the first temperature sensor 208 comprises a thermopile grid sensor.

FIGs. 4A-C depict the light-based skin treatment device 100 according to a second embodiment. In the second embodiment, the light-based skin treatment device 100 comprises a second temperature sensor 408. The second temperature sensor 408 is adapted to generate a second temperature signal representative of second temperature of a second portion 410 of the treatment area 206. The second temperature sensor 408 is adapted to sense the second temperature of the second portion 410 via the treatment aperture 110.

In FIG. 4A, the second portion 410 is represented as a point. In this embodiment, the second temperature sensor 408 is adapted to sense the temperature of only a small part of the treatment area 206, so the second portion 410 is small compared to the rest of the treatment area 206. In another embodiment, the second temperature sensor 408 is adapted to sense the temperature of a substantial portion of the treatment area 206, so the second portion 410 is a substantial part of the treatment area 206, such as 10% or 25% or 50% or more than 50% of the treatment area 206.

When the user operates the light-based skin treatment device 100, the light source 200 generates the treatment light 202. The treatment light 202 irradiates the treatment area 206 via the treatment aperture 110. As a result of the irradiation with the treatment light 202, the temperature of the treatment area 206 increases. As the second portion 410 is part of the treatment area 206, the second temperature signal senses the increase in temperature and generates the second temperature signal accordingly.

As shown in FIG. 4B, after the user operates the light-based skin treatment device 100 to irradiate the treatment area 206, the user moves the light-based skin treatment device 100 in the direction of arrow 212 towards a next portion of the skin that is to be treated. While the light-based skin treatment device 100 is moving, the second temperature sensor 408 senses the temperature of a portion 420 of the skin outside the treatment area 206 that was irradiated with the treatment light 202. The portion 420 has a lower temperature than the temperature of the first portion 210 in the irradiated treatment area 206, because the portion 420 has not yet been irradiated with the treatment light 202. For the same position, the first temperature sensor 208 still senses the higher temperature, as the first temperature sensor 208 still senses the temperature of the skin that was irradiated by the treatment light 202.

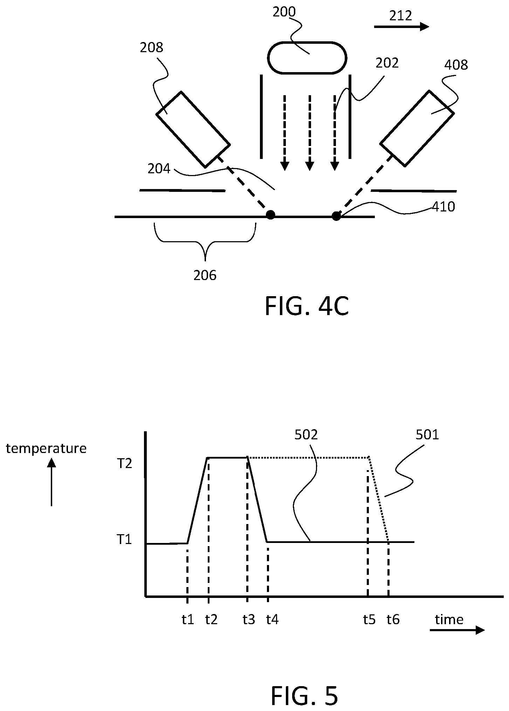

As shown in FIG. 4C, the user has moved the light-based skin treatment device 100 even further in the direction arrow 212 and arrived at the next portion of the skin that is to be treated. Both the first temperature sensor 208 and the second temperature sensor 408 sense temperatures of skin outside the treatment area 206 that was irradiated with treatment light 202.

FIG. 5 depicts the temperature as sensed by the first temperature sensor 208 and the second temperature sensor 408 according to the second embodiment. At the beginning, at time t0, the light-based skin treatment device 100 is held by the user at the treatment area 206, the first temperature sensor 208 senses the temperature of the first portion 210, and the second temperature sensor 408 senses the temperature of the second portion 410. At t0, the treatment area 206 is not yet irradiated with treatment light 202, so both the first temperature signal 501 and the second temperature signal 502 are representative of a low temperature T1. Then, around t1, the light source 200 starts to generate the treatment light 202, and the treatment area 206 is irradiated with the treatment light 202. As a result, the temperatures as sensed by the first temperature sensor 208 and the second temperature sensor 408 increase from the low temperature T1 at t1 to the higher temperature T2 at t2. After the treatment area 206 is irradiated with the treatment light 202, the light-based skin treatment device 100 is moved in the direction of arrow 212. Because of the direction of movement according to arrow 212, the second temperature sensor 408 starts to sense a temperature of skin outside the treatment area 206 at time t3, and at time t4, the second temperature sensor 408 senses the low temperature T1 of skin outside the treatment area 206. Meanwhile during time t3-t5, the first temperature sensor 208 remains directed to skin in the treatment area 206, and thus keeps on sensing the high temperature T2. At time t5, the first temperature sensor 208 starts to sense a temperature of skin outside the treatment area 206, and at time t6, the first temperature sensor 208 senses the low temperature T1 of skin outside the treatment area 206. From time t6, the first temperature sensor 208 and the second temperature sensor 408 sense the temperature of skin that is not irradiated by the treatment light 202, until the light source 200 generates the next irradiation with treatment light 202.

When both the first temperature signal 501 and the second temperature signal 502 indicate the low temperature T2, the treatment window faces untreated skin, so the light-based skin treatment device 100 is in a good position to provide treatment light 202, as overtreatment is prevented. In addition, the first temperature signal 501 and the second temperature signal 502 provide information about the direction of movement of the light-based skin treatment device 100, as the first temperature signal 501 and the second temperature signal 502 indicate that the second temperature sensor 408 is leading the movement, whereas the first temperature sensor 208 is trailing the movement.

Optionally, the first portion 210 comprises a first edge portion of the treatment area 206. The second portion 410 comprises a second edge portion of the treatment area 206. The second edge portion is opposite to the first edge portion.

Optionally, in the first embodiment or the second embodiment, at least one of the first portion 210 and the second portion 410 comprises a center portion of the treatment area 206. For example, the first portion 210 comprises a center portion of the treatment area 206, or the second portion 410 comprises a center portion of the treatment area 206. For example, the first portion 210 and the second portion 410 comprise a center portion of the treatment area 206, wherein the center portions are at least partly different from each other.

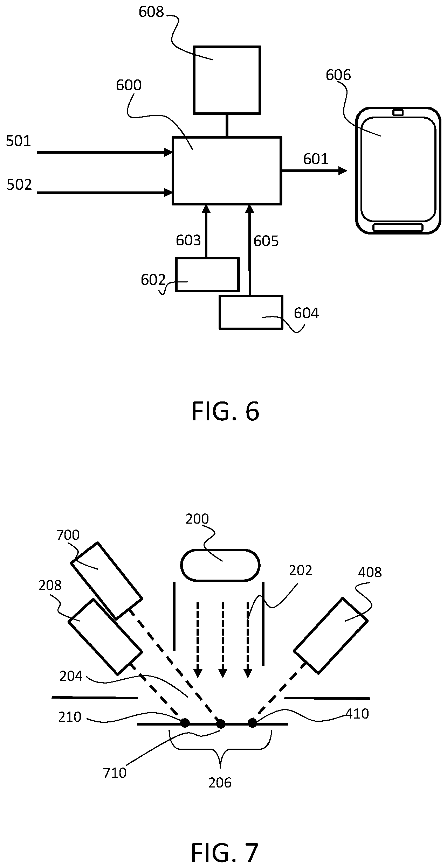

FIG. 6 depicts a processor 600 according to a third embodiment. The processor 600 according to the third embodiment is, for example, used in the light-based skin treatment device 100 according to the second embodiment.

In the third embodiment, the light-based skin treatment device 100 comprises the processor 600. The processor 600 is configured to generate, after irradiation of the treatment area 206 with the treatment light 202, an output signal 601 representative of a position of the light-based skin treatment device 100 relative to the skin based on the first temperature signal 501 and the second temperature signal 502.

Based on information about the measurement directions of the first temperature sensor 208 and the second temperature sensor 408, the processor 600 is able to determine the position based on how the first temperature signal 501 and the second temperature signal 502 change over time. For example, in case the first temperature signal 501 reduces from the high temperature T2 to the low temperature T1 before the second temperature signal 502 does, the light-based skin treatment device 100 is moving with the first temperature sensor 208 leading in a movement. For example, in case the second temperature signal 502 reduces from the high temperature T2 to the low temperature T1 before the second temperature signal 502 does, the light-based skin treatment device 100 is moving with the first temperature sensor 208 leading in the movement. The processor 600 determines the position based on the movement.

Optionally, the light-based skin treatment device 100 comprises a displacement sensor 602. The displacement sensor 602 is adapted to generate a displacement signal 603 representative of a displacement of the light-based skin treatment device 100 relative to the skin. The processor 600 is configured to determine the output signal 601 based on the displacement signal 603, the first temperature signal 501, and the second temperature signal 502. For example, the displacement sensor 602 is arranged behind the sensor window 114 and is arranged to sense displacement of the skin via the sensor window 114.

Optionally, the light-based skin treatment device 100 comprises a movement sensor 604. The movement sensor 604 is adapted to generate a movement signal 605 representative of a movement of the light-based skin treatment device 100. The processor 600 is configured to determine the output signal based on the movement signal 605, the first temperature signal 501, and the second temperature signal 502.

Optionally, the processor 600 is configured to control the light source 200 to generate a flash of treatment light 202 based on the first temperature signal 501 and the second temperature signal 502.

Optionally, the output signal 601 is adapted to cause a display 606 to display a graphical representation of the movement of the light-based skin treatment device 100. For example, the display 606 is of a mobile device.

For example, the processor 600 is in communication with a memory 608. The memory 608 comprises a computer program product having instructions. The instructions are executed by the processor 600.

FIG. 7 depicts a light-based skin treatment device 100 according to a fourth embodiment. The fourth embodiment has, for example, the same features as the first, or the second embodiment, except for the following. For example, the fourth embodiment includes the processor 600 according to the third embodiment.

In the fourth embodiment, the light-based skin treatment device 100 comprises a third temperature sensor 700 adapted to generate a third temperature signal representative of a third portion 710 of the treatment area 206. The third portion 710 of the treatment area 206 comprises a center portion of the treatment area 206. The first portion 210 comprises the first edge portion of the treatment area 206. The second portion 410 comprises a second edge portion of the treatment area 206, opposite to the first edge portion. So the third portion 710 is arranged in between the first portion 210 and the second portion 410.

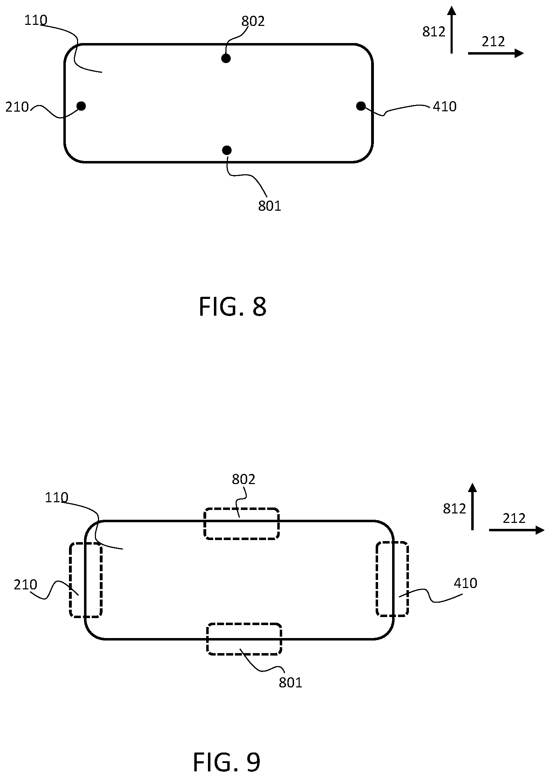

FIG. 8 depicts part of a light-based skin treatment device according to a fifth embodiment. The fifth embodiment has the same elements as, for example, the second embodiment or the fourth embodiment, except for the following.

FIG. 8 shows a front view of the treatment aperture 110. The first portion 210 is shown, where the first temperature sensor 208 senses a temperature. The second portion 410 is shown, where the second temperature sensor 408 senses a temperature. In addition to the first temperature sensor 208 and the second temperature sensor 408, the fifth embodiment has two additional temperature sensors, which are referred to as the fourth temperature sensor and the fifth temperature sensor. The fourth temperature sensor senses a temperature of the skin at a fourth portion 801 of the skin. The fifth temperature sensor senses a temperature of the skin at a fifth portion 802 of the skin. The fourth portion 801 and the fifth portion 802 are part of the treatment area 206.

The fourth temperature sensor and the fifth temperature sensor are arranged to have the fourth portion 801 and the fifth portion 802 at an offset from each other in a direction of arrow 812. The arrow 812 is perpendicular to the arrow 212. Due to this offset, if the user moves the light-based skin treatment device 100 in the direction of arrow 812, or in the opposite direction, the fourth temperature sensor and the fifth temperature sensor will move away from the treatment area 206 that was irradiated by the treatment light 202 at different times. For example, when moving in the direction of arrow 812, the fifth temperature sensor will face untreated skin, while the fourth temperature sensor still faces irradiated skin of the treatment area 206. So when moving in light-based skin treatment device 100 the direction of arrow 812, the fifth temperature sensor will sense a reduction of temperature at a different time than the fourth temperature sensor. Based on the information of the different time in sensing the reduction of temperature, the processor 600 is able to determine the position of the light-based skin treatment device 100.

As the processor 600 receives the temperature signals from the first temperature sensor 208, the second temperature sensor 408, the fourth temperature sensor, and the fifth temperature sensor, the processor 600 is able to determine the position of the light-based skin treatment device along two axes. These axes define a plane parallel to the skin facing the treatment aperture 110.

FIG. 9 depicts part of a light-based skin treatment device according to a sixth embodiment. The sixth embodiment has the same elements as, for example, the second embodiment or the fourth embodiment, or fifth embodiment except for the following.

In the sixth embodiment, the first temperature sensor 208 and the second temperature sensor 408 are arranged to sense temperature profiles over an area. The first temperature sensor 208 generates the first temperature signal 501 representative of a temperature profile of the first portion 210. The second temperature sensor 408 generates the second temperature signal 502 representative of a temperature profile of the second portion 410. Each of the first portion 210 and the second portion 410 is partly inside the treatment area 206 and partly outside the treatment area 206. So the first portion 210 comprises a first edge portion of the treatment area 206, and the second portion 410 comprises a second edge portion of the treatment area 206, opposite to the first edge portion. The temperature profiles represented by the first temperature signal 501 and the second temperature signal 502 indicate the high temperature T2 for the part that is inside the treatment area 206, and indicate the low temperature T1 for the part outside the treatment area 206. Based on the temperature profiles, the processor 600 is able to determine the edge of the treatment area 206 based on the first temperature signal 501 and the second temperature signal 502. This allows the processor 600 to determine the position of light-based skin treatment device 100 while moving in the direction of arrow 212 more accurately.

Optionally, the light-based skin treatment device 100 comprises the fourth temperature sensor and the fifth temperature sensor arranged to sense the temperature profiles over an area. The fourth temperature sensor generates a fourth temperature signal representative of a temperature profile of the fourth portion 801. The fifth temperature sensor generates the fifth temperature signal representative of a temperature profile of the fifth portion 802. Each of the fourth portion 801 and the fifth portion 802 is partly inside the treatment area 206 and partly outside the treatment area 206. So the fourth portion 801 comprises an edge portion of the treatment area 206, and the fifth portion 802 comprises an edge portion of the treatment area 206. The temperature profiles represented by the fourth temperature signal and the fifth temperature signal indicate the high temperature T2 for the part that is inside the treatment area 206, and indicate the low temperature T1 for the part outside the treatment area 206. Based on the temperature profiles, the processor 600 is able to determine the edge of the treatment area 206 based on the fourth temperature signal and the fifth temperature signal. This allows the processor 600 to determine the position of light-based skin treatment device 100 while moving in a plane defined by arrows 212 and 812 more accurately.

In the sixth embodiment, for example, one or more of the first temperature sensor 208, the second temperature sensor 408, the fourth temperature sensor, or the fifth temperature sensor comprises a thermal camera or a thermopile grid sensor.

FIG. 8 depicts a method according to a seventh embodiment. In the seventh embodiment, there is provided a computer program product. The computer program product comprises instructions, which when executed by the processor 600, cause a method to be carried out. The method comprises, in 1000, receiving the output signal 601 from the light-based skin treatment device 100 according to the third embodiment. The output signal 601 is adapted to cause a display to display the graphical representation of the position of the light-based skin treatment device 100. The method comprises, in 1001, causing the graphical representation representative of the position of the light-based skin treatment to be displayed on the display 606. The display 606 is, for example, on the mobile device. For example, the computer program product is stored in the memory 608.

In a setting at home, the user handling the light-based skin treatment device 100 places the head end 108 in contact with the user's own skin. In a commercial setting, such as a beauty salon or a spa, a professional user may handle the light-based skin treatment device 100 and may place the head end 108 in contact with the skin of another person, e.g., a client of the beauty salon.

For example, the method part of a non-therapeutic method of removing hairs with a light-based skin treatment device 100. For example, the method is part of a cosmetic method of removing hairs with a light-based skin treatment device 100. For example, the method is part of a non-therapeutic, cosmetic method of removing hairs with a light-based skin treatment device 100.

As discussed above, the processor 600 is adapted to perform the data processing. The processor 600 can be implemented in numerous ways, with software and/or hardware, to perform the various functions required. The processor 600 may employ one or more microprocessors that may be programmed using software (e.g. microcode) to perform the required functions. The processor 600 may be implemented as a combination of dedicated hardware to perform some functions and one or more programmed microprocessors and associated circuitry to perform other functions.

The processor 600 may include circuitry. Examples of circuitry that may be employed include, but are not limited to, conventional microprocessors, application specific integrated circuits (ASICs), and field-programmable gate arrays (FPGAs). Thus, the processor 600 may be embodied as a digital and/or analog processing system.

In various implementations, the processor 600 may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Functions implemented by a processor 600 may be implemented by a single processor 600 or by multiple separate processing units which may together be considered to constitute a "processor". Such processing units may in some cases be remote from each other and communicate with each other in a wired or wireless manner.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A light-based skin treatment device (100) comprising:
a light source (200) adapted to generate treatment light (202);
a treatment aperture (110) arranged to allow passage of the treatment light (202) to irradiate a treatment area (206) of skin with the treatment light (202);
a first temperature sensor (208) adapted to generate a first temperature signal (501) representative of a first temperature of a first portion (210) of the treatment area (206);
wherein the first temperature sensor (208) is adapted to sense the first temperature of the first portion (210) via the treatment aperture (110).

2. The light-based skin treatment device (100) according to claim 1,
wherein the first temperature sensor (208) is arranged outside a path of the treatment light (202).

3. The light-based skin treatment device (100) according to claim 1 or 2,
wherein the first temperature sensor (208) is arranged to receive the electromagnetic radiation from the first portion (210) via a radiation path (214) through the treatment aperture (110).

4. The light-based skin treatment device (100) according to any one of the preceding claims, comprising
a second temperature sensor (408) adapted to generate a second temperature signal (502) representative of second temperature of a second portion (410) of the treatment area (206),
wherein the second temperature sensor (408) is adapted to sense the second temperature of the second portion (410) via the treatment aperture (110).

5. The light-based skin treatment device (100) according to claim 4, comprising a processor (600) configured to generate, after irradiation of the treatment area (206) with the treatment light (202), an output signal (601) representative of a position of the light-based skin treatment device (100) relative to the skin based on the first temperature signal (501) and the second temperature signal (502).

6. The light-based skin treatment device (100) according to claim 5, comprising
a displacement sensor (602) adapted to generate a displacement signal (603) representative of a displacement of the light-based skin treatment device (100) relative to the skin,
wherein the processor (600) is configured to determine the output signal (601) based on the displacement signal (603), the first temperature signal (501), and the second temperature signal (502).

7. The light-based skin treatment device (100) according to claim 5 or 6, comprising
a movement sensor (604) adapted to generate a movement signal (605) representative of a movement of the light-based skin treatment device (100),
wherein the processor (600) is configured to generate the output signal (601) based on the movement signal (605), the first temperature signal (501), and the second temperature signal (502).

8. The light-based skin treatment device (100) according to any one of claims 5-7,
wherein the processor (600) is configured to control the light source (200) to generate a flash of treatment light (202) based on the first temperature signal (501) and the second temperature signal (502).

9. The light-based skin treatment device (100) according to any one of claims 5-8,
wherein the output signal (601) is adapted to cause a display (606) to display a graphical representation of the position of the light-based skin treatment device (100).

10. The light-based skin treatment device (100) according to claims 4-9,
wherein at least one of the first portion (210) and the second portion (410) comprises a center portion of the treatment area (206).

11. The light-based skin treatment device (100) according to claims 4-9,
wherein the first portion (210) comprises a first edge portion of the treatment area (206),
wherein the second portion (410) comprises a second edge portion of the treatment area (206), opposite to the first edge portion.

12. The light-based skin treatment device (100) according to claim 11, comprising a third temperature sensor (700) adapted to generate a third temperature signal representative of a third portion (710) of the treatment area (206),
wherein the third portion (710) of the treatment area (206) comprises a center portion of the treatment area (206).

13. The light-based skin treatment device (100) according to any one of the preceding claims,
wherein the first temperature sensor (208) comprises an infrared sensor or a radiance sensor.

14. The light-based skin treatment device (100) according to any one of the preceding claims, wherein the light-based skin treatment device is an Intense Pulsed Light device adapted for photo-epilation.

15. A computer program product comprising instructions, which when executed by a processor (600), cause a method to be carried out, the method comprising:
receiving (1000) the output signal (601) from the light-based skin treatment device (100) according to any one of claims 5-9;
causing (1001) the graphical representation representative of the position of the light-based skin treatment device (100) to be displayed on the display (606).
